# EUROPEAN PATENT APPLICATION

(11) **EP 4 403 149 A1**
(43) Date of publication of application: **24.07.2024**
(21) Application number: 22910994.7
(22) Date of filing: 12.12.2022
(51) Int. Cl.: A61F 13/15, A61F 13/53, A61F 13/539

(54) **ABSORBENT ARTICLE**

(30) Priority: 20.12.2021 JP 2021206501
(71) Applicant: Unicharm Corporation, Ehime 799-0111 (JP)
(72) Inventor: BANDOU, Takeshi, Kanonji-shi, Kagawa 769-1602 (JP); MURAI, Takamasa, Kanonji-shi, Kagawa 769-1602 (JP); WATANABE, Sakiko, Kanonji-shi, Kagawa 769-1602 (JP); MIYAZAKI, Hirokazu, Kanonji-shi, Kagawa 769-1602 (JP); TODA, Haruki, Kanonji-shi, Kagawa 769-1602 (JP)
(74) Representative: Dolleymores
(86) International application number: PCT/JP2022/045736
(87) International publication number: WO 2023/120286

(57) **Abstract**

This absorbent article is characterized by comprising: a recycled part (33a) which has a vertical direction, a transverse direction, and a thickness direction orthogonal to each other and which is composed of recycled pulp obtained by recycling used disposable absorbent articles; and sheet members (38, 34, 37) which each overlap at least a portion of the recycled part (33a) when viewed in the thickness direction. This absorbent article is characterized in that the recycled part (33a) contains a recycling-derived component, while the sheet members (38, 34, 37) are disposed closer to the skin side as compared with the recycled part (33a).

## Description

### FIELD

The present invention relates to an absorbent article.

### BACKGROUND

For the purpose of suppressing the consumption of limited resources and reducing the burden on the environment, Patent Document 1 describes the collection of recycled paper that contains pulp from absorbent articles such as disposable diapers, for example.

### [CITATION LIST]

### [PATENT LITERATURE]

[Patent Document 1] Japanese Patent Application Publication No. 2021-75819

### SUMMARY

### [TECHNICAL PROBLEM]

Like the absorbent article in Patent Document 1, in the case where recycled paper is collected from used absorbent articles and the collected recycled paper is used for newly-manufacturing absorbent articles, the recycled paper contain components associated with recycling, causing a risk of negatively influencing on the wearer's skin.

The present invention was achieved in light of conventional problems such as that described above, an aspect of the present invention is to provide an absorbent article that reduces a risk of direct contact between the wearer's skin and a recycled part which contains a recycled-derived component.

### [SOLUTION TO PROBLEM]

A main aspect of the present invention of achieving the above-described aspect is an absorbent article having a longitudinal direction, a lateral direction, and a thickness direction that are orthogonal to each other, the absorbent article including: a recycled part containing recycled pulp that has been obtained by making a used disposable absorbent article be subject to a recycling process; and a sheet member that overlaps at least a part of the recycled part as viewed in the thickness direction, the recycled part containing a recycled-derived component, the sheet member being provided on a skin side with respect to the recycled part. Features of the present invention other than the above will become clear by reading the description of the present specification with reference to the accompanying drawings.

### [ADVANTAGEOUS EFFECT OF THE INVENTION]

According to the present invention, it is possible to reduce negative effects of a recycled-derived component on the skin.

### BRIEF DESCRIPTION OF DRAWINGS

FIG. 1 is a schematic plan view of an absorbent pad 1 in an unfolded and stretched state, as viewed from a skin surface side.
FIG. 2 is a sectional view taken along the line A-A in FIG. 1.
FIG. 3 is a sectional view taken along the line B-B in FIG. 1.
FIG. 4 is a diagram illustrating an adhesive portion 38s.
FIG. 5 is a schematic plan view of the disposable diaper 100 in the unfolded and stretched state, as viewed from the skin surface side.
FIG. 6 is a sectional view taken along the line D-D in FIG. 5.
FIG. 7 is a diagram illustrating an adhesive portion 104s and an adhesive portion 107s.

### DESCRIPTION OF EMBODIMENTS

At least the following matters will be clear with the description of this specification and the attached drawings.
An absorbent article having a longitudinal direction, a lateral direction, and a thickness direction that are orthogonal to each other, the absorbent article including: a recycled part containing recycled pulp that has been obtained by making a used disposable absorbent article be subject to a recycling process; and a sheet member that overlaps at least a part of the recycled part as viewed in the thickness direction, the recycled part containing a recycled-derived component, the sheet member being provided on a skin side with respect to the recycled part.

According to the absorbent article, the sheet member reduces the risk of direct contact between the wearer's skin and the recycled part which contains recycled-derived component, and makes it possible to reduce negative effects of the recycled-derived component contained in the recycled part, on the skin.

In such an absorbent article, it is desirable that the recycled part is an absorbent core that absorbs liquid, that the absorbent core has liquid absorbent pulp, that the liquid absorbent pulp has the recycled pulp that has at least the recycled-derived component, and that a pH of an aqueous solution that is obtained by adding 100 mL of an ion exchange water to 10 g of the liquid absorbent pulp is 5.5 or less.

According to the absorbent article, even in the case where the absorbent core in which the pH is 5.5 or less is used, the sheet member provided on the skin side with respect to the absorbent core makes it possible to reduce a risk that the absorbent core and the wearer's skin come into contact, and to reduce a risk that excrement which has been absorbed by the absorbent core return to the wearer's skin with increased acidity.

In such an absorbent article, it is desirable that the pH of the aqueous solution that is obtained by adding 100 mL of the ion exchange water to 10 g of the liquid absorbent pulp is between 3.0 and 5.0 (inclusive).

According to the absorbent article, the sheet member that is provided on the skin side with respect to the absorbent core reduces the negative effects on the wearer's skin and whereas, even in the case where the pH of the absorbent core is 5.0 or less and there is a risk of having negative effect on the wearer's skin, if the pH of the absorbent core is 3.0 or higher, the alkaline of the excrement makes it likely to neutralize the acidity of the liquid absorbent pulp of the absorbent core during usage, making it possible to reduce negative effects on the wearer's skin.

In such an absorbent article, it is desirable that the recycled part is a core-wrapping sheet that covers at least a part of an absorbent core that absorbs liquid, and that a pH of an aqueous solution that is obtained by adding 100 mL of an ion exchange water to 10 g of the core-wrapping sheet is 5.5 or less.

According to the absorbent article, even in the case where the core-wrapping sheet in which the pH is 5.5 or less is used, the sheet member provided on the skin side with respect to the core-wrapping sheet makes it possible to reduce a risk that the absorbent core and the wearer's skin come into contact, and contacting with the core-wrapping sheet makes it possible to reduce a risk that excrement which has been absorbed by the absorbent core return to the wearer's skin with increased acidity.

In such an absorbent article, it is desirable that the pH of the aqueous solution that is obtained by adding 100 mL of the ion exchange water to 10 g of the core-wrapping sheet is between 3.0 and 5.0 (inclusive).

According to the absorbent article, the sheet member that is provided on the skin side with respect to the core-wrapping sheet reduces the negative effects on the wearer's skin and whereas, even in the case where the pH of the core-wrapping sheet is 5.0 or less and there is a risk of having negative effect on the wearer's skin, if the pH of the core-wrapping sheet is 3.0 or higher, the alkaline of the excrement makes it likely to neutralize the acidity of the liquid absorbent pulp of the core-wrapping sheet e during usage, making it possible to reduce negative effects on the wearer's skin.

In such an absorbent article, it is desirable that the recycled-derived component is either of a sulfuric acid and a hypochlorous acid.

According to the absorbent article, even in the case where the recycled part has sulfuric acid or hypochlorous acid as the recycled-derived component, the sheet member provided on the skin side with respect to the recycled part can reduce a risk that the recycled part and the wearer's skin come into contact, and can reduce a risk that the excrement which has been absorbed once by the recycled part returns to the wearer's skin with increased acidity due to contact with recycled-derived component.

In such an absorbent article, it is desirable that the recycled part is an absorbent core that absorbs liquid, that the absorbent core has liquid absorbent pulp, that the liquid absorbent pulp has the recycled pulp that has at least the recycled-derived component, and that a pH of an aqueous solution that is obtained by adding 100 mL of an ion exchange water to 10 g of the liquid absorbent pulp is greater than 7.0.

According to the absorbent article, even in the case where the absorbent core in which the pH is greater than 7.0 is used, the sheet member provided on the skin side with respect to the absorbent core makes it possible to reduce a risk that the absorbent core and the wearer's skin come into contact, and to reduce a risk that excrement which has been absorbed by the absorbent core return to the wearer's skin with increased alkalinity.

In such an absorbent article, it is desirable that the absorbent core also has superabsorbent polymer containing sodium acrylate.

According to the absorbent article, when the absorbent core absorbs moisture from the excrement and the like during usage, the acrylic acid group generated by the release of the sodium acrylate is made likely to neutralize the alkaline of the liquid absorbent pulp, making it possible to reduce negative effects on the wearer's skin.

In such an absorbent article, it is desirable that the recycled part is a core-wrapping sheet that covers at least a part of an absorbent core that absorbs liquid, and that a pH of an aqueous solution that is obtained by adding 100 mL of an ion exchange water to 10 g of the core-wrapping sheet is 7.0 or more.

According to the absorbent article, even in the case where the core-wrapping sheet in which the pH is 7.0 or more is used, the sheet member provided on the skin side with respect to the core-wrapping sheet makes it possible to reduce a risk that the absorbent core and the wearer's skin come into contact, and contacting with the core-wrapping sheet makes it possible to reduce a risk that excrement which has been absorbed by the absorbent core return to the wearer's skin with increased alkalinity.

In such an absorbent article, it is desirable that the recycled-derived component is a nitrogen compound, and that a weight of nitrogen within the nitrogen compound in the recycled part is greater than 0 ppm and equal to or less than 50 ppm.

According to the absorbent article, even in the case where the recycled part containing the nitrogen compound is used, making the weight ratio of nitrogen within the nitrogen compound be equal to or less than 50 ppm makes it possible to reduce negative effects on the wearer's skin compared to the case where the weight ratio of nitrogen within the nitrogen compound is greater than 50 ppm. In addition, due to the sheet member provided on the skin side with respect to the recycled part, it is possible to reduce the risk of contact between the recycled part and the wearer's skin, and it is possible to reduce the risk of negative effects on the wearer's skin even if the excrement that has been absorbed once by the absorbent article returns to the wearer's skin side with increased alkalinity.

In such an absorbent article, it is desirable that the recycled-derived component is either of calcium chloride and a nitrogen compound.

According to the absorbent article, in the case where the core-wrapping sheet has the recycled-derived component of either of calcium chloride and the nitrogen compound, the sheet member provided on the skin side with respect to the core-wrapping sheet can reduce a risk that the core-wrapping sheet and the wearer's skin come into contact, and also it is possible to reduce a risk that the excrement that has been absorbed once by the absorbent core returns to the wearer's skin with increased alkalinity due to contact with the liquid absorbent pulp.

In such an absorbent article, it is desirable that an adhesive is provided in a non-skin-side surface of the sheet member, and that the adhesive is hydrophobic.

According to the absorbent article, it is possible to reduce the risk that the excrement that has been absorbed by the absorbent article during usage and that has once reached to the recycled part returns to the wearer's skin.

In such an absorbent article, it is desirable that either of followings is satisfied: both ends of the adhesive in the longitudinal direction are match both ends of the sheet member in the longitudinal direction; and both ends of the adhesive in the lateral direction match both ends of the sheet member in the lateral direction.

According to the absorbent article, it is possible to further reduce the risk that the excrement that has been absorbed by the absorbent article during usage and that has once reached to the recycled part returns to the wearer's skin.

In such an absorbent article, it is desirable that the sheet member is made of a thermoplastic resin fiber, and that the sheet member is provided so as to be able to come into contact with a wearer's skin.

According to the absorbent article, the sheet member that is made of the thermoplastic resin fiber is provided in a position where it comes into contact with the wearer's skin, and therefore even if the excrement that has been absorbed by the absorbent article during usage and has once reached the recycled part returns to the wearer's skin, it reduces the risk of the sheet member retaining the excrement, making it likely to reduce the risk of the recycled-derived component coming into contact with the wearer's skin.

In such an absorbent article, it is desirable that the sheet member includes a liquid-permeable sheet portion and a liquid-impermeable sheet portion.

According to the absorbent article, this allows the liquid-permeable sheet portion to absorb the excrement, and also by providing the liquid-impermeable sheet portion, it is possible to reduce the risk that the excrement which has been absorbed by the absorbent article during usage and has once reached the recycled part returns to the wearer's skin.

In such an absorbent article, it is desirable that, at a center in the longitudinal direction, lateral side ends of the sheet member are located outside corresponding lateral side ends of the recycled part.

According to the absorbent article, at the center of the absorbent article in the longitudinal direction, the sheet member can cover the recycled part from the skin side at least in the lateral direction, and this makes it possible to reduce the risk that the recycled part comes into direct contact with the wearer's skin.

In such an absorbent article, it is desirable that the recycled-derived component has a sodium polyacrylate a molecular weight of which is less than 500.

According to the absorbent article, although the sodium polyacrylate whose molecular weight is less than 500 penetrates into the wearer's skin and has a negative effect on the wearer's skin, the sheet member provided on the skin side with respect to the recycled part can reduce the risk of the recycled-derived component coming into contact with the wearer's skin. In addition, it is possible to reduce the risk that the excrement that has been absorbed once by the absorbent core returns to the wearer's skin in a state of containing the recycled-derived component due to contact with the liquid absorbent pulp.

### Embodiments

For the purpose of suppressing the consumption of limited resources and reducing the burden on the environment, it is desired to recycle disposable absorbent articles and manufacture new absorbent articles. The absorbent article according to the present invention includes pulp that has been obtained from the recycling process with used disposable absorbent article (hereinafter also referred to as the "recycled pulp"). The recycled pulp is one that is recovered by collecting disposable absorbent articles that have been used and making them be subject to the recycling process. By using recycled parts that contains this recycled pulp, it is possible to new absorbent articles.

The recycled part contains recycled-derived components depending on the nature of the members used by to-recycle absorbent articles, the usage conditions of to-recycle absorbent articles, methods of the recycling process an the like. For example, the recycled-derived components may permeate the inside of the recycled pulp of the recycled part, or may adhere to the surface of the recycled pulp of the recycled part.

The "recycled-derived component" refers to the components included in the recycled pulp that has been recovered by making used absorbent articles be subject to the recycling process as follow: components included in the absorbent article itself regardless of being used or unused and excluding pulp that has not undergone recycling; components of excrement that has been absorbed by used absorbent articles (during usage); components obtained due to making used absorbent articles be subject to the recycling process; and the like. Examples of the "components included in the absorbent article itself regardless of being used or unused and excluding pulp that has not undergone recycling" include components such as plastic, superabsorbent polymer (SAP), and the like, that are used for the absorbent article before recycling. Examples of "components of excrement that has been absorbed by used absorbent articles (during usage)" include components derived from the excrement such as feces, urine, and blood that have been absorbed during usage of the absorbent article. Examples of "components obtained due to making used absorbent articles be subject to the recycling process" include components resulting from cleaning and processing used absorbent articles. This recycled-derived component is a component the pulp which has not undergone recycling does not contain (hereinafter also referred to as "virgin pulp").

An absorbent article that is newly manufactured using the recycled pulp has a risk that recycled-derived components negatively influences on the wearer's skin. In the following embodiments, an absorbent article including a recycled part will be described.

### First embodiment

The following describes an absorbent pad 1 that absorbs excrement such as urine and feces by way of example of an absorbent article according to the first embodiment.

### Basic structure of the absorbent pad 1

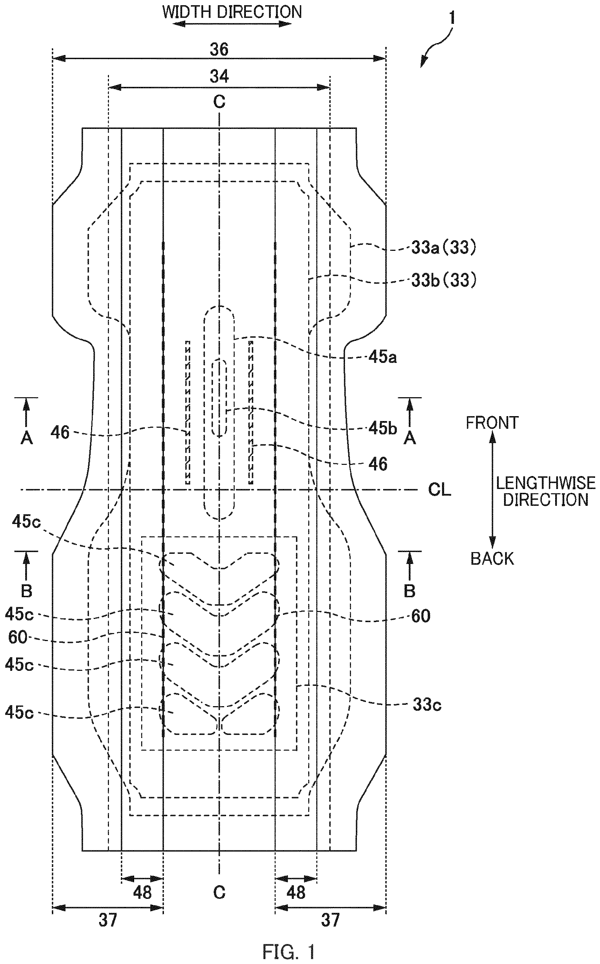

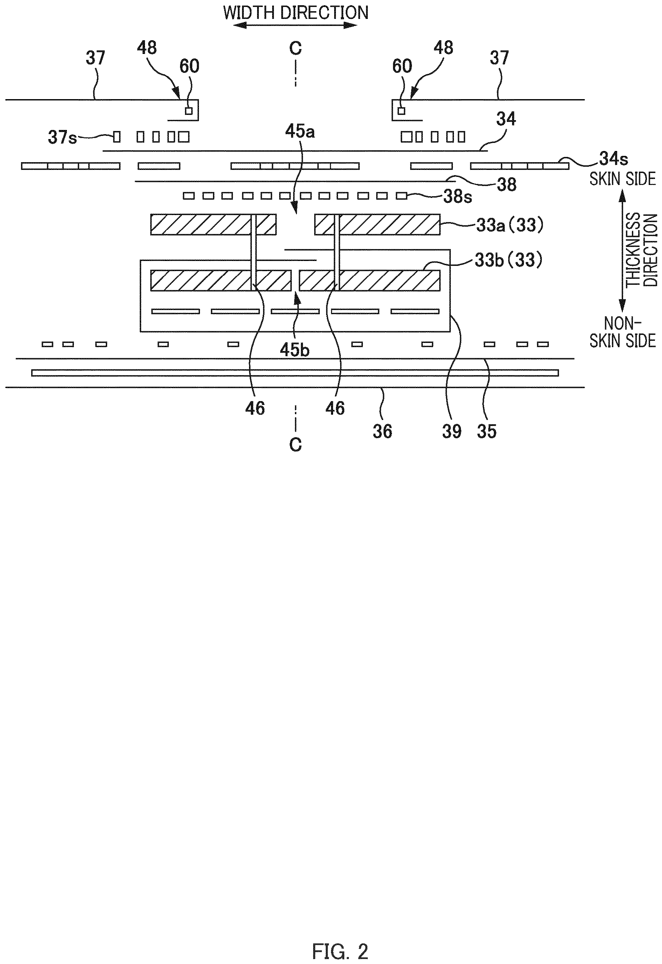

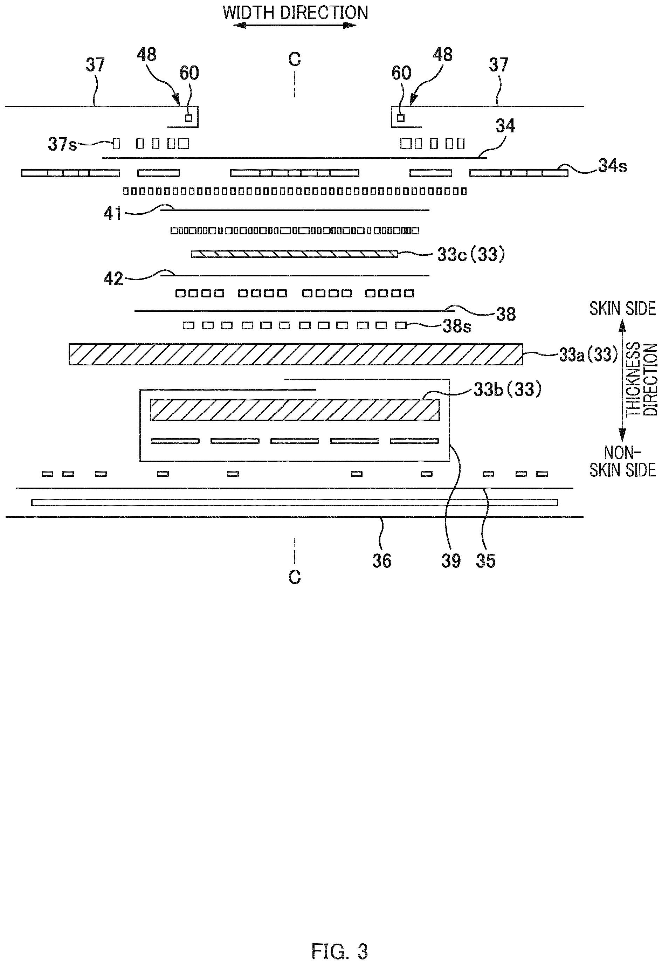

FIG. 1 is a schematic plan view of the absorbent pad 1 in an unfolded and stretched state, which is an absorbent article according to the first embodiment, as viewed from the skin surface side. FIG. 2 is a sectional view taken along the line A-A in FIG. 1. FIG. 3 is a sectional view taken along the line B-B in FIG. 1. In FIG. 1, etc., the center line CC is the center line in the width direction in the stretched state, and the center line CL is the center line in the lengthwise direction in the stretched state.

As shown in FIGS. 1 to 3, the absorbent pad 1 has a lengthwise direction, a width direction, and a thickness direction. The direction in which the constituent members of the absorbent pad 1 are overlaid on each other is referred to as the thickness direction. In the thickness direction, the side that is in contact with the wearer is called the skin side, and the opposite side is called the non-skin side. The lengthwise direction of the absorbent pad 1 is also referred to as the longitudinal direction. In the lengthwise direction, the side provided on the wearer's stomach side is the front side, and the side provided on the wearer's back side is the back side. The width direction of the absorbent pad 1 is also referred to as the left-right direction, and the absorbent pad 1 of the present embodiment has a configuration symmetrical to the width direction.

The unfolded state of the absorbent pad 1 refers to a state in which the absorbent pad 1 is opened in the lengthwise direction and thus the absorbent pad 1 is unfolded in a plan view. In addition, the stretched state of the absorbent pad 1 refers to a state in which the entire absorbent pad 1 (entire product) has been stretched to eliminate wrinkles, or more specifically, the absorbent pad 1 has been stretched with not being affected by the elastic members included in the absorbent pad 1 until the dimensions of the constituent materials of the absorbent pad 1 match or is close to the dimensions of the material on their own.

As shown in FIGS. 1 to 3, the absorbent pad 1 includes a top-surface sheet 34, a back-surface sheet 35, an absorbent body 33, an exterior sheet 36, and a pair of side sheets 37. The top-surface sheet 34 is a liquid-permeable sheet provided on the skin side in the thickness direction with respect to the absorbent body 33, and examples thereof include an air-through nonwoven fabric sheet, a spunbond nonwoven fabric sheet, and the like. The back-surface sheet 35 is a liquid-impermeable sheet provided on the non-skin side in the thickness direction with respect to the absorbent body 33, and examples thereof include a synthetic resin film and a hydrophobic SMS nonwoven fabric sheet and the like. Further, it is preferable that the top-surface sheet 34 and the back-surface sheet 35 contain thermoplastic resin (polyethylene (PE), polypropylene (PP), and the like).

The pair of side sheets 37 each are a sheet (e.g., nonwoven fabric sheet, and the like) located on the skin side of the top-surface sheet 34 and extending outward in the width direction from both side portions in the width direction. The pair of side sheets 37 contain thermoplastic resin (polyethylene (PE), polypropylene (PP), and the like). The exterior sheet 36 is a liquid-impermeable sheet located on the non-skin side in the thickness direction with respect to the back-surface sheet 35, and has a shape (that is, the external shape of the absorbent pad 1) in which the center portion in the lengthwise direction is narrowed inward in the width direction.

The absorbent body 33 is a member that is located between the top-surface sheet 34 and the back-surface sheet 35 in the thickness direction and that has liquid absorbency and liquid retaining properties. In the present embodiment, the absorbent body 33 includes a first absorbent core 33a located on the skin side, a second absorbent core 33b located on the non-skin side of the first absorbent core 33a, and a third absorbent core 33c located on the skin side of the first absorbent core 33a.

For example, the first absorbent core 33a and the second absorbent core 33b can be obtained by molding liquid-absorbent pulp containing superabsorbent polymer (SAP) into a predetermined shape. Specifically, in the present embodiment, the shape of the first absorbent core 33a is a substantially gourd shape or a substantially hourglass shape, which is elongated in the lengthwise direction and has a portion narrowed inward in the width direction, near the center in the lengthwise direction. On the other hand, the shape of the second absorbent core 33b has a substantially rectangular shape which is long in the lengthwise direction and short in the width direction in a plan view. In the second absorbent core 33b, the dimension in the lengthwise direction is shorter than the dimension of the first absorbent core 33a in the lengthwise direction, and the dimension in the width direction is shorter than the dimension of the first absorbent core 33a in the width direction. Therefore, both edges of the first absorbent core 33a in the lengthwise direction and the width direction are respectively located outside both edges of the second absorbent core 33b in the lengthwise direction and the width direction.

Examples of the third absorbent core 33c include an SAP sheet shaped into a layer-form by attaching SAP (superabsorbent polymer) to a hydrophilic sheet. The third absorbent core 33c of the present embodiment has a substantially rectangular shape that is elongated in the lengthwise direction, and that is provided in the center in the width direction and on the back side in the lengthwise direction of the absorbent pad 1. Specifically, the third absorbent core 33c is provided on the back side in the lengthwise direction with respect to a slit 45a (described later). Note that there is no particular limitation on the shape of the absorbent body 33.

Further, in the present embodiment, a first core-wrapping sheet 38 is provided on the skin side of the first absorbent core 33a, and a second core-wrapping sheet 39 is provided so as to surround the second absorbent core 33b from the non-skin side. Further, a third core-wrapping sheet 41 is provided on the skin side of the third absorbent core 33c, and a fourth core-wrapping sheet 42 is provided on the non-skin side of the third absorbent core 33c. The core-wrapping sheets 38, 39, 41, and 42 may contains a thermoplastic resin in addition to the pulp fiber as the liquid-absorbent fiber. Examples of the thermoplastic resin include polyethylene (PE) and polypropylene (PP).

Further, the absorbent pad 1 has leak-proof walls 48. The leak-proof walls 48 are configured to be able to rise up on the side of the wearer's skin surface, and are provided in pair on the left and right sides. As shown in FIGS. 2 and 3, the leak-proof walls 48 are each formed as follow: an inner side portion of the side sheet 37 in the width direction is folded back to the non-skin side, and a leak-proof-wall elastic member 60 that stretches and contracts in the lengthwise direction is provided in the folded back portion. The leak-proof wall 48 can suppress the side leakage of excrement. In addition, in FIG. 1, one string-like leak-proof-wall elastic member 60 is arranged in the width direction, but the number and arrangement of the leak-proof-wall elastic members 60 is not particularly limited.

In the present embodiment, in the absorbent body 33, the first absorbent core 33a has a slit 45a that is recessed, preferably penetrating, in the thickness direction from the skin side towards the non-skin side. The slit 45a is located on the front side with respect to the center of the absorbent pad 1 in the lengthwise direction, and extends in the lengthwise direction through the center of the absorbent body 33 in the width direction. The second absorbent core 33b has a slit 45b that is recessed, preferably penetrating, in the thickness direction from the skin side towards the non-skin side. The slit 45b is located on the back side with respect to the center of the absorbent pad 1 in the lengthwise direction, and extends in the lengthwise direction through the center of the absorbent body 33 in the width direction. The slit 45a and the slit 45b are formed so that at least portions thereof overlap in a plan view. Thereby, when excreted urine reaches the second absorbent core 33b via the slit 45a of the first absorbent core 33a, the urine can be drawn further into the second absorbent core 33b via the slit 45b. The dimensions (length) of the slits 45a and 45b in the lengthwise direction and the dimensions (width) of the slits 45a and 45b in the width direction in a plan view are adjusted as appropriate in consideration of the sizes of the first absorbent core 33a and the second absorbent core 33b. The width and length of the slits 45a and 45b are, for example, 5.0 to 50 mm and 50 to 300 mm.

The third absorbent core 33c of the present embodiment has been compressed in the thickness direction, and the uncompressed portion of the third absorbent core 33c protrudes toward the skin side as a protrusion 45c.

In the present embodiment, the absorbent body 33 includes a pair of compressed portions 46. The pair of compressed portions 46 extend along the lengthwise direction on both outer sides of the slit 45a in the width direction. The pair of the compressed portions 46 are formed by compressing the first absorbent core 33a and the second absorbent core 33b in the thickness direction from the skin-side surface of the first absorbent core 33a. However, the pair of compressed portions 46 may be formed by compressing both the top-surface sheet 34 and the absorbent body 33. The pair of compressed portions 46 are each formed in a straight-line manner in a plan view, but the shape is not particularly limited. The pair of the compressed portions 46 can prevent urine from moving outward on the top-surface sheet 34 and leaking to the outside, and can make it likely to draw urine into the absorbent body 33.

### Recycled part of absorbent pad 1

The absorbent pad 1 includes the absorbent cores 33a and 33b, which have the recycled pulp, as the recycled part. In other words, the absorbent cores 33a and 33b of the absorbent pad 1 are the recycled part that include the recycled pulp obtained by making used disposable absorbent articles (e.g., used disposable diapers) be subject to the recycling process. Although the absorbent pad 1 of the present embodiment includes the first absorbent core 33a and the second absorbent core 33b as the recycled part, the recycled part provided in the absorbent pad 1 may be only the first absorbent core 33a or only the second absorbent core 33b. Although as the recycled part, the absorbent core 33a will be described, but the same applies to the absorbent core 33b as the recycled part.

The first absorbent core 33a of the absorbent pad 1 has the recycled pulp, which has been obtained by making used disposable absorbent articles be subject to the recycling process. As mentioned above, the first absorbent core 33a is obtained by molding liquid absorbent pulp containing superabsorbent polymer (SAP) into a predetermined shape. The entirety of the liquid absorbent pulp may be the recycled pulp, or the liquid absorbent pulp may be obtained by mixing the recycled pulp with the virgin pulp in any ratio.

There is a risk that the recycled-derived component included in the recycled part negatively influence on the wearer's skin. Therefore, as shown in FIGS. 1 to 3, the absorbent pad 1 has the first core-wrapping sheet 38 as a "sheet member" that overlaps at least a part of the first absorbent core 33a, which is the recycled part, as viewed in the thickness direction (in the plan view). The first core-wrapping sheet 38 is provided on the skin side with respect to than the first absorbent core 33a, which is the recycled part, and is provided between the wearer's skin and the first absorbent core 33a. In addition, the "sheet member" (first core-wrapping sheet 38) provided on the skin side with respect to the recycled part (first absorbent core 33a) is a sheet-like member that does not contain recycled-derived components, or is also a sheet-like member that contains a small amount of recycled-derived components than the recycled part (first absorbent core 33a).

As a result, a portion where the first absorbent core 33a and the first core-wrapping sheet 38 overlap as viewed in the thickness direction can reduce the risk that the wearer's skin comes into direct contact with the first absorbent core 33a, making it possible to reduce the risk that the recycled-derived component contained the first absorbent core 33a negatively influence the wearer's skin.

As mentioned above, the first absorbent core 33a, which is the recycled part, has the liquid absorbent pulp containing the recycled pulp. The ratio of the recycled pulp and the virgin pulp in the liquid absorbent pulp of the first absorbent core 33a can be set arbitrarily. In addition, the liquid absorbent pulp in the first absorbent core 33a is pulp fiber obtained by removing superabsorbent polymer (SAP) etc. from the first absorbent core 33a, and in the case of using the virgin pulp, it is the combination of the recycled pulp and the virgin pulp.

The pH of the aqueous solution that is obtained by adding 100 mL of the ion exchange water to 10 g of the liquid absorbent pulp of the first absorbent core 33a may be 5.5 or less. That is, the liquid absorbent pulp of the first absorbent core 33a, which has the recycled-derived component, may has acidic properties. Even in the case where the pH of the aqueous solution that is obtained by adding 100 mL of the ion exchange water to 10 g of the liquid absorbent pulp is 5.5 or less and there is a possibility of having negative effect on the wearer's skin, by using the first core-wrapping sheet 38 provided on the skin side with respect to the first absorbent core 33a, it is possible to reduce the risk of direct contact between the first absorbent core 33a and the wearer's skin. Further, during usage, there is a risk that the excrement that has been absorbed by the first absorbent core 33a returns to the wearer's skin. At this time, there is a risk that the excrement in which the acidity has been increased by the liquid absorbent pulp of the first absorbent core 33a returns to the skin side. In this regard, by providing the first core-wrapping sheet 38 on the skin side with respect to the first absorbent core 33a, this makes it easier to reduce the amount of the excrement that returns to the wearer's skin from the first absorbent core 33a, making it possible to reduce the risk that the excrement with increased acidity comes into contact with the wearer's skin.

The pH of the aqueous solution that is obtained by adding 100 mL of the ion exchange water to 10 g of the liquid absorbent pulp of the first absorbent core 33a may be between 3.0 and 5.0 (inclusive). Even in such a case, the first core-wrapping sheet 38 provided on a skin side with respect to the first absorbent core 33a can reduce the risk that the wearer's skin come into direct contact with the first absorbent core 33a. In addition, even in the case where the pH of the aqueous solution that is obtained by adding 100 mL of the ion exchange water to 10 g of the liquid absorbent pulp of the first absorbent core 33a is 5.0 or less and there is a possibility of having negative effect on the wearer's skin, if the pH is 3.0 or more, the alkaline included in the excrement that has been absorbed during usage makes it likely to neutralize the acidity of the liquid absorbent pulp, making it likely to reduce the negative effects on the wearer's skin.

In addition, regarding the excrement which has been absorbed by the absorbent pad 1, the feces generally shows alkaline due to the influence of digestive juices, and the like. In addition, urine shows weak acidity, and as time lapses in a state of being absorbed by the absorbent pad 1, it changes to the alkaline. Therefore, the excrement which has been absorbed by the absorbent pad 1 generally shows alkaline in most cases. However, the acidity and the alkalinity of the excrement may change depending on constitution, medical condition, diet, living environment, etc. of the wearer.

The pH value of liquid absorbent pulp can be measured in the following method. First, the first absorbent core 33a is taken out from the absorbent pad 1 that is in a dry state (unused state), and superabsorbent polymer (SAP) is removed from the first absorbent core 33a by a well-known method, resulting in the state of the liquid absorbent pulp. Subsequently, 100 mL of water (the ion exchange water is added to 10 g of the liquid absorbent pulp in an Erlenmeyer flask, and the flask is placed at a room temperature of 20 to 25°C for 1 hour in a state where 10 g of the liquid absorbent pulp is impregnated with water. During this time period, the Erlenmeyer flask is shaken as appropriate. Then, the obtained extract is filtered through a glass filter, potassium chloride (2mL) is added to the filtrate, and the resulting mixture is measured with a pH meter to obtain the pH value.

The pH value of the liquid absorbent pulp of the first absorbent core 33a may be measured for all the liquid absorbent pulp of the first absorbent core 33a, or for a part of the liquid absorbent pulp of the first absorbent core 33a. In addition, the pH value is measured by adding 100mL of the ion exchange water to 10 g of the liquid absorbent pulp. For example, if the weight of all the liquid absorbent pulp included in the first absorbent core 33a is 5 g, the pH value of the aqueous solution to which 50mL of the ion exchange water is added is measured.

Further, the recycled-derived component of the first absorbent core 33a, which is the recycled part, has may be sulfuric acid or hypochlorous acid. These sulfuric acid or hypochlorous acid are components contained in the recycled pulp mainly because of being subjected to the recycling process. Even if the first absorbent core 33a has sulfuric acid or hypochlorous acid as a recycled-derived component, the first core-wrapping sheet 38 provided on the skin side with respect to the first absorbent core 33a can reduce a risk of direct contact between the first absorbent core 33a and the wearer's skin. In addition, due to the first core-wrapping sheet 38 provided on the skin side with respect to the first absorbent core 33a, it is possible to reduce a risk that the excrement that has been absorbed once by the first absorbent core 33a during usage returns to the skin side with the acidity of the excrement increased due to the liquid absorbent pulp of the first absorbent core 33a.

As mentioned above, the first absorbent core 33a, which is the recycled part, has the liquid absorbent pulp containing the recycled pulp, and the liquid absorbent pulp is pulp fiber obtained by removing superabsorbent polymer (SAP) etc. from the first absorbent core 33a. In the case of using the virgin pulp, the liquid absorbent pulp is the combination of the recycled pulp and the virgin pulp. The pH of the aqueous solution that is obtained by adding 100 mL of the ion exchange water to 10 g of the aqueous solution of the first absorbent core 33a may be greater than 7.0. Even in the case where the pH of the aqueous solution that is obtained by adding 100 mL of the ion exchange water to 10 g of the liquid absorbent pulp is greater than 7.0, that is, even in the case where 10 g of the liquid absorbent pulp has the alkaline properties and has negative effect on the wearer's skin, the first core-wrapping sheet 38 provided on a skin side with respect to the first absorbent core 33a makes it possible to reduce the risk of direct contact between first absorbent core 33a and the wearer's skin. In addition, during usage, there is a risk that the excrement that has been absorbed by the first absorbent core 33a returns from the first absorbent core 33a toward the skin side, and at this time, there is a risk that the excrement in which the alkalinity has been increased by the liquid absorbent pulp of the first absorbent core 33a returns to the skin side. So, the first core-wrapping sheet 38, which is provided on the skin side with respect to the first absorbent core 33a, makes it easier to reduce the amount of the excrement that moves toward the skin side from the first absorbent core 33a, making it possible to reduce the risk that the excrement with increased alkalinity comes into contact with the wearer's skin. The pH value of liquid absorbent pulp can be measured in the same manner as described above.

The recycled-derived component included in the first absorbent core 33a, which is the recycled part, may be calcium chloride or a nitrogen compound. Calcium chloride or the nitrogen compound are components contained in the recycled pulp or components derived from the excrement mainly because of being subject to the recycling process. Even if the first absorbent core 33a has calcium chloride or a nitrogen compound as a recycled-derived component, the first core-wrapping sheet 38 provided on the skin side with respect to the first absorbent core 33a can reduce a risk that direct contact between the first absorbent core 33a and the wearer's skin. In addition, due to the first core-wrapping sheet 38 provided on the skin side with respect to the first absorbent core 33a, it is possible to reduce a risk that the excrement that has been absorbed once by the first absorbent core 33a during usage returns to the skin side with the alkalinity of the excrement increased due to the liquid absorbent pulp of the first absorbent core 33a.

In the case where the pH of the aqueous solution that is obtained by adding 100 mL of the ion exchange water to 10 g of the liquid absorbent pulp of the first absorbent core 33a is greater than 7.0, it is preferable that the first absorbent core 33a also has superabsorbent polymer containing sodium acrylate. The first absorbent core 33a of the absorbent pad 1 of the present embodiment has superabsorbent polymer (SAP) composed of sodium polyacrylate, in addition to the liquid absorbent pulp containing the recycled pulp. This sodium polyacrylate shows weak acidity. Therefore, when the first absorbent core 33a absorbs moisture from the excrement and the like during usage, the acrylic acid group generated by the release of the sodium acrylate can neutralize the alkaline of the liquid absorbent pulp.

For example, there is shown below the neutralization reaction in the case where the recycled-derived component of the first absorbent core 33a (liquid absorbent pulp) is calcium chloride and where the pH of the aqueous solution that is obtained by adding 100 mL of the ion exchange water to 10 g of the liquid absorbent pulp is greater than 7.0.

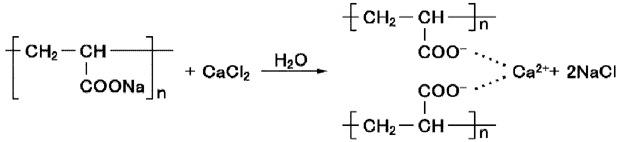

Even in the case where the liquid absorbent pulp of the first absorbent core 33a shows the alkaline due to the recycled-derived component, having sodium polyacrylate (superabsorbent polymer) which shows acidity makes it likely to facilitate neutralization in the presence of moisture, and this weakens the alkaline of the liquid absorbent pulp, making it likely to reduce the negative effect on the wearer's skin.

In addition, in the case where the recycled-derived component contained in the first absorbent core 33a (liquid absorbent pulp), which is the recycled part, is a nitrogen compound, it is preferable that the weight of nitrogen within the nitrogen compound in the first absorbent core 33a (recycled part) is greater than 0 ppm and equal to or less than 50 ppm. In the case where the recycled-derived component is a nitrogen compound, the nitrogen compound is a component derived from the excrement that has been absorbed by the absorbent article before recycling. For example, it is decomposed products derived from proteins and/or amino acids, such as ammonia, that is, components derived from the excrement that has been absorbed by the absorbent article before recycling. The pH value of the liquid absorbent pulp that contains nitrogen compounds as a recycled-derived component shows the alkaline (pH greater than 7). Even in the case where the first absorbent core 33a has a nitrogen compound as a recycled-derived component, when the weight of nitrogen within the nitrogen compound in the first absorbent core 33a is greater than 0 ppm and equal to or less than 50 ppm, it is possible to reduce negative effects on the wearer's skin compared to the case where the weight ratio of nitrogen within the nitrogen compound is greater than 50 ppm. In addition, the first core-wrapping sheet 38 that is provided on the skin side with respect to the first absorbent core 33a reduces the risk of direct contact between the first absorbent core 33a and the wearer's skin. Simultaneously, even in the case where the alkalinity of the excrement that has been absorbed once by the first absorbent core 33a is increased due to the first absorbent core 33a (liquid absorbent pulp) that contains the nitrogen compound, the absorbed excrement is less likely to return to the skin side, making it possible to reduce the risk of negative effects on the wearer's skin.

The weight ratio of nitrogen within the nitrogen compound to the first absorbent core 33a (recycled part) is obtained in a known method. For example, in order to determine the nitrogen content of the nitrogen compound relative to the first absorbent core 33a, which is the recycled part, the weight of the first absorbent core 33a is measured first. Then, remove the superabsorbent polymer (SAP) which does not contain nitrogen, in the first absorbent core 33a. In the state where the superabsorbent polymer (SAP) is removed the first absorbent core 33a, leaving only the liquid absorbent pulp, the nitrogen content is measured using the Kjeldahl method or a method such as a trace nitrogen meter, and the weight of the obtained nitrogen is compared with the weight of the first absorbent core 33a. By doing this, the weight ratio of nitrogen within the nitrogen compound to the first absorbent core 33a (recycled part) is obtained.

The recycled-derived component contained in the first absorbent core 33a, which is the recycled part, may be a decomposed product of superabsorbent polymer (SAP), the molecular weight of the decomposed product being less than 500, and in particular, may be sodium polyacrylate whose molecular weight is less than 500. In the case where the recycled-derived component is sodium polyacrylate (decomposed product of SAP) whose molecular weight is less than 500, the sodium polyacrylate (decomposed product of SAP) whose molecular weight is less than 500 is a decomposed product of the superabsorbent polymer (SAP) that is used in the absorbent article before recycling. It is sodium polyacrylate whose molecular weight has become less than 500 due to the decomposition of the superabsorbent polymer (SAP) in the recycling process of used absorbent articles.

In the field of dermatology, it is known that components having a molecular weight of less than 500 is likely to penetrate into the skin (the so-called "500 Dalton rule"). Therefore, if the recycled part contains sodium polyacrylate (decomposed product of SAP) whose molecular weight is less than 500, the sodium polyacrylate penetrates into the skin of the wearer who puts on the absorbent article including the recycled part, causing a risk of negative effects on the wearer's skin. In this regard, in the absorbent pad 1, by providing the first core-wrapping sheet 38 on the skin side with respect to the first absorbent core 33a, which is the recycled part, this reduces the risk of direct contact between the first absorbent core 33a and the wearer's skin, making it possible to reduce the risk that the sodium polyacrylate whose molecular weight is less than 500 penetrates into the wearer's skin. In addition, even in the case where the excrement that has been absorbed once by the first absorbent core 33a contains the sodium polyacrylate whose molecular weight is less than 500, it makes the absorbed excrement less likely to return to the skin side, and makes it possible to reduce the risk of negative effects on the wearer's skin.

Regarding the first core-wrapping sheet 38 provided on the skin side with respect to the first absorbent core 33a, which is the recycled part, it is preferable that the adhesive portion 38s where an adhesive is applied is provided to the non-skin-side surface of the first core-wrapping sheet 38 and that the adhesive is hydrophobic. Also, regarding this hydrophobic adhesive, it is more preferable that both ends of the adhesive in the lengthwise direction match both ends of the first core-wrapping sheet 38 in the lengthwise direction, or both ends of the adhesive in the width direction match both ends of the first core-wrapping sheet 38 in the width direction.

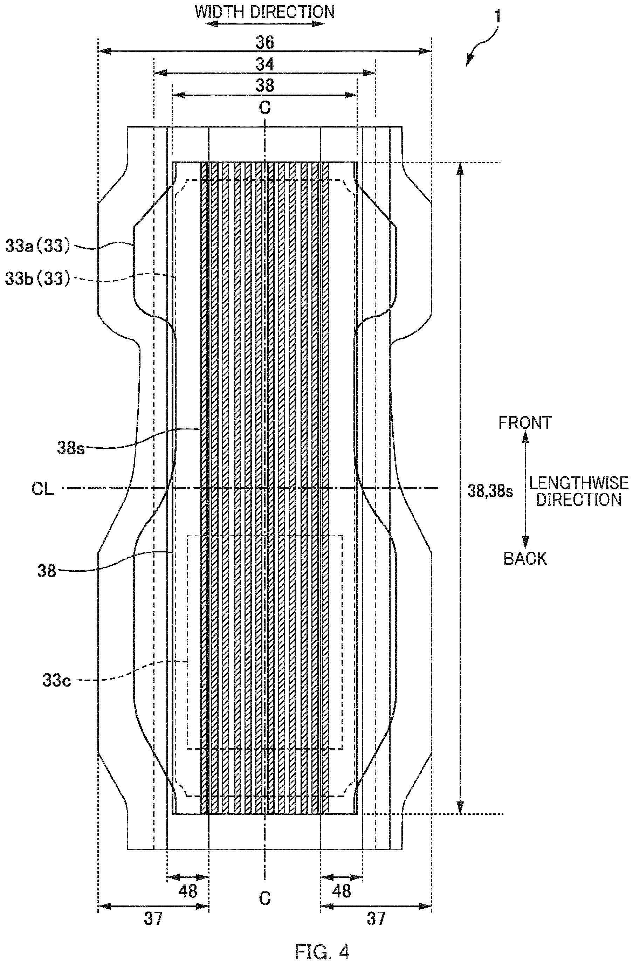

FIG. 4 is a diagram illustrating the adhesive portion 38s provided in the non-skin-side surface of the first core-wrapping sheet 38. For convenience, in FIG. 4, the first absorbent core 33a, the first core-wrapping sheet 38, and the adhesive portion 38s are shown with solid lines, and the slits 45a and 45b, the compressed portion 46, and the like are omitted.

The absorbent pad 1 of the present embodiment has the adhesive portion 38s in which an adhesive is applied to the non-skin-side surface of the first core-wrapping sheet 38 and the first core-wrapping sheet 38 and the first absorbent core 33a adhere to each other. As shown in FIGS. 2 to 4, the adhesive portion 38s has a plurality of strip-shaped (rectangular) adhesive regions arranged side-by-side in the width direction, and both ends of the adhesive portion 38s in the lengthwise direction match both ends of the first core-wrapping sheet 38 in the lengthwise direction, and match both ends of the first absorbent core 33a in the lengthwise direction. Further, both widthwise ends of the adhesive portion 38s are provided inside both widthwise ends of the first core-wrapping sheet 38 and inside both widthwise ends of the first absorbent core 33a.

Even in the case where the excrement that has been absorbed by the absorbent pad 1 during usage reaches the first absorbent core 33a, the hydrophobic adhesive provided between the first core-wrapping sheet 38 and the first absorbent core 33a makes it likely to suppress the excrement that is in contact with the recycled-derived component in the first absorbent core 33a from returning to the wearer's skin. In addition, the larger the area of the adhesive provided in the non-skin-side surface of the first core-wrapping sheet 38, the more the amount of the excrement returning to the wearer's skin from the first absorbent core 33a can be reduced. Accordingly, the following configuration is more preferable: both lengthwise ends of the adhesive provided in the non-skin-side surface of the first core-wrapping sheet 38 match both lengthwise ends of the first core-wrapping sheet 38; or both widthwise ends of the adhesive match both widthwise ends of the first core-wrapping sheet 38.

Note that the application pattern of the adhesive in the adhesive portion 38s can be changed arbitrarily. The absorbent pad 1 of the present embodiment has an application pattern with so-called stripes elongated in the lengthwise direction, but the application pattern may also be the stripe pattern with stripes elongated in the lateral direction, an omega pattern, or the like. Or, the adhesive may also be applied in a predetermined region inside the first core-wrapping sheet 38 without gaps.

Furthermore, the sheet member that is provided on the skin side with respect to the first absorbent core 33a, which is the recycled part, is not limited to the first core-wrapping sheet 38. It is sufficient that the sheet member is a sheet-like member that does not have the recycled-derived component or is a sheet-like member that has less recycled-derived component than the recycled part (first absorbent core 33a) has, and that the sheet member is provided on the skin side in the thickness direction with respect to the first absorbent core 33a.

In the absorbent pad 1 of the present embodiment, a configuration is acceptable in which the top-surface sheet 34 or the side sheets 37 are provided on the skin side with respect to the first absorbent core 33a, as shown in FIG. 2 and the like. The top-surface sheet 34 and the side sheets 37 each overlap at least a part of the first absorbent core 33a, which is the recycled part, when viewed in the thickness direction and these sheets 34 and 37 are provided on the skin side with respect to the first absorbent core 33a. Therefore, it is possible to reduce a risk that the wearer's skin comes into direct contact with the first absorbent core 33a, and this makes it possible to reduce the risk that the recycled-derived component contained the first absorbent core 33a negatively influence the wearer's skin.

The following configuration is preferable: the top-surface sheet 34 and the side sheets 37 are provided so as to be able to come into contact with the wearer's skin in a put-on state; and the top-surface sheet 34 and the side sheets 37 are each made of nonwoven fabric, etc., and made of thermoplastic resin fiber (e.g., polyethylene (PE) and polypropylene (PP),). By making the top-surface sheet 34 and the side sheets 37 that are made of thermoplastic resin fiber be able to come into contact with the skin in the put-on state, even if the excrement that has been absorbed by the absorbent pad 1 during usage and has once reached the first absorbent core 33a returns to the wearer's skin, it is possible to reduce the risk that the top-surface sheet 34 and the side sheets 37 retain the excrement and thereby the excrement remains in contact with the wearer's skin. This makes it likely to reduce the risk that the excrement containing the recycled-derived component remains in contact with the wearer's skin.

Furthermore, if the sheet member provided on a skin side with respect to the first absorbent core 33a is made of the thermoplastic resin fiber and can come into contact with the wearer's skin, it is preferable that the sheet member includes a liquid-permeable sheet and a liquid-impermeable sheet. The absorbent pad 1 includes the liquid-permeable top-surface sheet 34 and the liquid-impermeable side sheets 37, as the sheet members which are provided on the skin side with respect to the first absorbent core 33a. This allows the liquid-permeable top-surface sheet 34 to absorb the excrement. In addition, by providing the liquid-impermeable side sheets 37, even if the excrement has been absorbed by the absorbent pad 1 during usage and has once reached the first absorbent core 33a, it is possible to reduce the risk that the excrement containing recycled-derived component returns to the wearer's skin.

In addition, it is preferable that, at the center in the lengthwise direction (center line CL), the widthwise side ends of the sheet member of the sheet member provided on the skin side with respect to the first absorbent core 33a, which is the recycled part, are respectively located outside the widthwise side ends of the first absorbent core 33a. In the absorbent pad 1 of the present embodiment, the widthwise side ends of the top-surface sheet 34 are located outside the widthwise side ends of the first absorbent core 33a, on the center line CL in the lengthwise direction. That is, in the width direction, the first absorbent core 33a is provided inside the top-surface sheet 34. As a result, at least in the width direction, the first absorbent core 33a, which is the recycled part, can be completely covered from the skin side, and this makes it likely to reduce the risk that the first absorbent core 33a containing the recycled-derived component comes into direct contact with the wearer's skin.

Furthermore, the sheet member provided on a skin side with respect to the first absorbent core 33a, which is the recycled part, may be a sheet-like layer formed by superabsorbent polymer (so-called SAP). In the absorbent pad 1, the third absorbent core 33c may be the sheet member. As shown in FIGS. 1 and 3, the third absorbent core 33c overlaps a part (part on the back side) of the first absorbent core 33a, which is the recycled part, when viewed in of the thickness direction, and the third absorbent core 33c is provided on the skin side with respect to the first absorbent core 33a. Further, the third absorbent core 33c is a sheet-like member that do not have the recycled-derived component or have fewer recycled-derived component than the first absorbent core 33a. Note that, as the third absorbent core 33c, it is possible to use a sheet formed by adhering SAP particles to nonwoven fabric or the like, or a sheet formed only of SAP.

With the third absorbent core 33c, a portion where the first absorbent core 33a and the third absorbent core overlap as viewed in the thickness direction can reduce the risk that the wearer's skin come into direct contact with the first absorbent core 33a, and this makes it possible to reduce the risk that recycled-derived component contained in the first absorbent core 33a has negative effects on the wearer's skin.

Further, in the case where the superabsorbent polymer is sodium polyacrylate, the third absorbent core 33c has weak acidic properties. Therefore, in the case where the liquid absorbent pulp of the first absorbent core 33a has alkaline properties due to the recycled-derived component, it is preferable that the third absorbent core 33c composed of sodium polyacrylate is the sheet member. During usage of the absorbent core 1, when the first absorbent core 33a absorbs moisture from the excrement and the like, the acrylic acid group generated by the release of the sodium acrylate can neutralize the alkaline of the liquid absorbent pulp. In other words, even in the case where the liquid absorbent pulp of the first absorbent core 33a shows the alkaline due to the recycled-derived component, having sodium polyacrylate (superabsorbent polymer) which shows acidity makes it likely to facilitate neutralization in the presence of moisture, and this weakens the alkaline of the liquid absorbent pulp, making it likely to reduce the negative effect on the wearer's skin.

As shown in FIGS. 2 and 3, the adhesive portion 34s and the adhesive portions 37s are provided with the adhesive applied to the skin-side surfaces of the top-surface sheet 34 and the skin-side surfaces of the side sheets 37, respectively. The adhesive portion 34s is mainly provided between the top-surface sheet 34 and the first core-wrapping sheet 38, and the adhesive portions 37s are mainly provided between the side sheets 37 and the top-surface sheet 34. These adhesive portions are portions where the members is made adhere and fixed. It is preferable that the adhesive is a region where a hydrophobic adhesive is applied, similar to the adhesive portion 38s of the first core-wrapping sheet 38.

### Second embodiment

The following describes a tape-type disposable diaper for adults (hereinafter also referred to as a diaper 100) by way of example of an absorbent article according to the second embodiment.

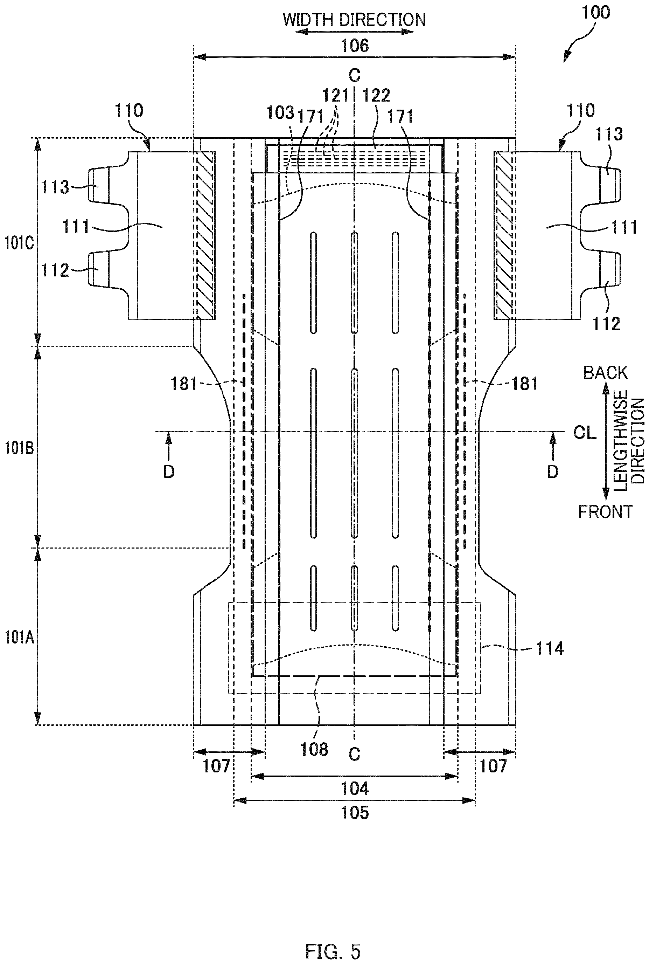

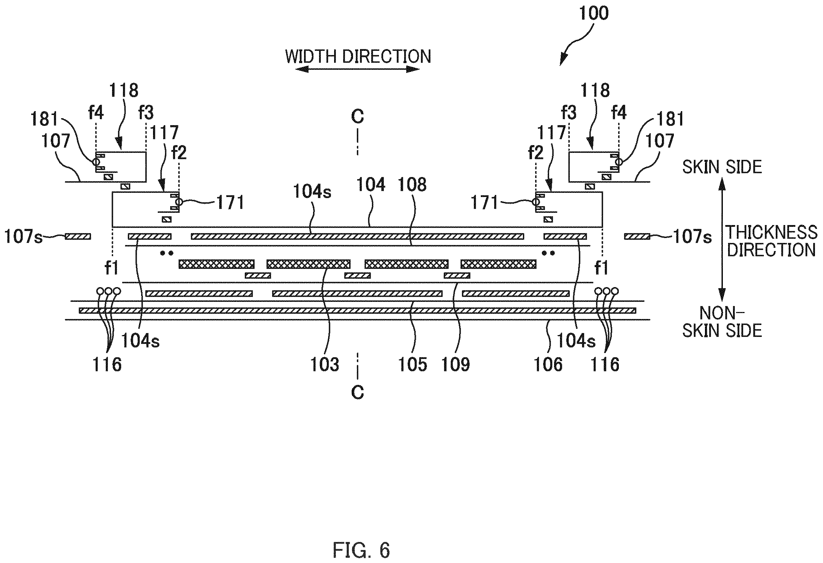

FIG. 5 is a schematic plan view of the disposable diaper 100 in the unfolded and stretched state, which is an absorbent article according to the second embodiment, as viewed from the skin surface side. FIG. 6 is a sectional view taken along the line D-D in FIG. 5.

As shown in FIG. 5, the diaper 100 has the lengthwise direction, the width direction, and the thickness direction the unfolded and stretched state. In addition, the diaper 100 is divided into three regions in the lengthwise direction, and includes: a front waist portion 101A that is applied to the wearer's stomach side; a back waist portion 101C that is applied to the wearer's back side; and a crotch portion 101B that is located between front waist portion 101A and the back waist portion 101C in the lengthwise direction. Further, as shown in FIG. 6, the direction in which the constituent members of the diaper 100 are overlaid on each other is referred to as the thickness direction. In the thickness direction, the side that is in contact with the wearer is called the skin side, and the opposite side is called the non-skin side. The lengthwise direction of the diaper 100 is also referred to as the longitudinal direction. In the lengthwise direction, the side provided on the wearer's stomach side is the front side, and the side provided on the wearer's back side is the back side. The width direction of the diaper 100 is also referred to as the left-right direction, and the diaper 100 of the present embodiment has a symmetrical configuration to the width direction. Note that the unfolded state and the stretched state of the diaper 100 are the same as the definitions of the unfolded state and the stretched state of the absorbent pad 1 in the first embodiment.

As shown in FIG. 6, the diaper 100 includes: an absorbent core 103; a top-surface sheet 104 provided on the skin side in the thickness direction with respect to the absorbent core 103; a back-surface sheet 105 and an exterior sheet 106 that are provided on the non-skin side in the thickness direction with respect to the absorbent core 103, and a pair of side sheets 107.

The top-surface sheet 104 only needs to be a liquid-permeable sheet, and examples thereof include an air-through nonwoven fabric sheet, a spunbond nonwoven fabric sheet and the like. The back-surface sheet 105 only needs to be a liquid-impermeable sheet, and examples thereof include a synthetic resin film and a hydrophobic SMS nonwoven fabric sheet and the like. Additionally, it is preferable that the top-surface sheet 104 and the back-surface sheet 105 contain thermoplastic resin (polyethylene (PE), polypropylene (PP), and the like).

The pair of side sheets 107 each are a sheet (e.g., nonwoven fabric sheet, and the like) located on the skin side of the top-surface sheet 104 and extending outward in the width direction from both side portions in the width direction. The exterior sheet 106 is a liquid-impermeable sheet located on the non-skin side in the thickness direction with respect to the back-surface sheet 105, and has a shape (that is, the external shape of the diaper 100) in which the center portion (crotch portion 101B) in the lengthwise direction is narrowed inward in the width direction.

For example, the absorbent core 103 can be obtained by molding liquid-absorbent fibers (e.g., pulp) containing SAP (superabsorbent polymer) into a predetermined shape. In addition, of the present embodiment, a liquid-permeable skin-side core-wrapping sheet 108 is arranged adjacent to the skin side of the absorbent core 103, and a liquid-permeable non-skin-side core-wrapping sheet 109 is arranged adjacent to the non-skin side of the absorbent core 103. The skin-side core-wrapping sheet 108 and the non-skin-side core-wrapping sheet 109 are constituted by tissue paper, for example, and the details thereof will be described later.

The diaper 100 also includes flap portions 110 extending outward in the width direction from the back waist portion 101C, as shown in FIG. 5. The flap portions 110 each include a base sheet 111, and a first fastening member 112 and a second fastening member 113 that are joined to the base sheet 111. The base sheet 111 is joined between the side sheet 107 and the exterior sheet 106. The first fastening member 112 and the second fastening member 113 are arranged spacing in the lengthwise direction from each other, and are configured to be able to fasten to a target portion 114 when putting on the diaper 100, the target portion 114 being provided in the front waist portion 101A. The first fastening member 112 and the second fastening member 113 can be a hook material, for example. Note that a configuration may be acceptable in which one fastening member is provided on each of the left and right sides. Moreover, a configuration may be acceptable in which the diaper 100 does not include the base sheet 111 of the flap portion 110, but the side sheet 107 and the exterior sheet 106 extend outward in the width direction, and the fastening members are joined onto the side sheet 107.

The diaper 100 has leg elastic members 116 that stretches and contracts in the lengthwise direction on both side portions in the width direction (see FIG. 6). Three leg elastic members 116 are provided on each side in the width direction with spacing from each other. More specifically, the leg elastic member 116 that is located farthest inside in the width direction is arranged between the top-surface sheet 104 and the back-surface sheet 105. The remaining two leg elastic members 116 are arranged between the side sheet 107 and the back-surface sheet 105. Note that the number of the leg elastic members 116 is not particularly limited.

Moreover, the diaper 100 has inner leak-proof gathers 117 and outer leak-proof gathers 118. The inner leak-proof gathers 117 and the outer leak-proof gathers 118 are configured to be able to rise up on the side of the wearer's skin surface, and are provided in pair on the left and right sides. The inner leak-proof gathers 117 are so-called inward-folding gathers, and the outer leak-proof gathers 118 are so-called outward-folding gathers. The inner leak-proof gathers 117 are each provided inside the corresponding outer leak-proof gather 118 and leg elastic members 116 in the width direction. Note that it is sufficient that at least a part of the inner leak-proof gather 117 is located inside the corresponding outer leak-proof gather 118 in the width direction. Note that the diaper 100 does not necessarily need to have the inner leak-proof gathers 17 and the outer leak-proof gathers 118, and may have only the outer leak-proof gathers 118.

The inner leak-proof gather 117 is formed by folding the top-surface sheet 104, as shown in FIG. 6. Specifically, the inner leak-proof gather 117 is formed as follow: the top-surface sheet 104 is folded inward from the outside in the width direction and toward the skin side at a folding line f1 shown in FIG. 6, and further the top-surface sheet 104 is folded back outward from the inside in the width direction and toward the non-skin side at a folding line f2, and an inner leak-proof elastic member 171 that stretches and contracts in the lengthwise direction is provided in the folded-back portion. Examples of the inner leak-proof elastic member 171 include elastic strings.

The outer leak-proof gather 118 is formed by folding the side sheet 107, as shown in FIG. 6. Specifically, the outer leak-proof gather 118 is formed as follow: the side sheet 107 is folded outward from the inside in the width direction and toward the skin side at the folding line f3 shown in FIG. 2, and further the side sheet 107 is folded back inward from the outside in the width direction and toward the non-skin side at the folding line f4, and an outer leak-proof elastic member 181 that stretches and contracts in the lengthwise direction is provided in the folded-back portion. Examples of the outer leak-proof elastic member 181 include elastic strings. The inner leak-proof gathers 117 and the outer leak-proof gathers 118 can suppress lateral leakage of excrement.

In the back waist portion 101C of the diaper 100, a waist gather 122 formed by a plurality of waist elastic members 121 is arranged in the end in the lengthwise direction and in the center in the width direction. Due to the waist gather 122, the diaper 1 deforms to fit the shape of the wearer's waist when putting on, and also is suppressed diaper 100 from shifting.

### recycled part of diaper 100

The diaper 100 includes the skin-side core-wrapping sheet 108 and the non-skin-side core-wrapping sheet 109 that contain the recycled pulp as the recycled part. That is, the skin-side core-wrapping sheet 108 and the non-skin-side core-wrapping sheet 109 of the diaper 100 are a recycled part containing recycled pulp that is obtained by recycling pulp collected from used disposable absorbent articles (e.g., used disposable diapers). In addition, although the diaper 100 of the present embodiment includes the skin-side core-wrapping sheet 108 and the non-skin-side core-wrapping sheet 109 as the recycled part, the recycled part provided in the diaper 100 may be only the skin-side core-wrapping sheet 108 or only the non-skin-side core-wrapping sheet 109. Hereinafter, as the recycled part, the skin-side core-wrapping sheet 108 will be described, but the same applies to the non-skin-side core-wrapping sheet 109.

The skin-side core-wrapping sheet 108 is so-called tissue that is made from the recycled pulp collected from used absorbent articles as a raw material and formed into a sheet by adding additives such as a thickener. The pulp contained in the skin-side core-wrapping sheet 108 may be only the recycled pulp, or may be obtained by mixing the recycled pulp with the virgin pulp in any ratio.

There is a risk that the recycled-derived component included in the recycled part negatively influence on the wearer's skin. Therefore, as shown in FIGS. 5 and 6, the diaper 100 has the top-surface sheet 104 as a "sheet member" that overlaps at least a part of the skin-side core-wrapping sheet 108, which is the recycled part the thickness direction (in plan view). The top-surface sheet 104 is provided on the skin side with respect to the recycled part, and is provided between the wearer's skin and the skin-side core-wrapping sheet 108. In addition, the "sheet member" (top-surface sheet 104) provided on the skin side with respect to the recycled part (skin-side core-wrapping sheet 108) is a sheet-like member that does not contain recycled-derived components, or is also a sheet-like member that contains a small amount of recycled-derived components than the recycled part (skin-side core-wrapping sheet 108).

As a result, a portion where the skin-side core-wrapping sheet 108 and the top-surface sheet 104 overlap as viewed in the thickness direction can reduce the risk that the wearer's skin comes into direct contact with the skin-side core-wrapping sheet 108, making it possible to reduce the risk that the recycled-derived component contained the skin-side core-wrapping sheet 108 negatively influence the wearer's skin.

In addition, in the diaper 100 of the present embodiment, since the sheet member provided on the skin side with respect to the skin-side core-wrapping sheet 108 is the top-surface sheet 104, the risk that the wearer's skin comes into direct contact with the skin-side core-wrapping sheet 108, which is the recycled part, can be reduced without increasing the number of materials included in the diaper 100. Note that the top-surface sheet 104 can come into contact with the wearer's skin while being put on, and is preferably made of thermoplastic resin fiber (polyethylene (PE), polypropylene (PP), etc.) such as nonwoven fabric. As a result, in the case where if the excrement that has been absorbed by the absorbent pad 1 during usage and has once reached the skin-side core-wrapping sheet 108 returns to the wearer's skin, it is possible to reduce the risk that the top-surface sheet 104 that is in contact with the wearer's skin retains the excrement and thereby the excrement remains in contact with the wearer's skin. This makes it likely to reduce the risk that the excrement containing the recycled-derived component remains in contact with the wearer's skin.

Regarding the top-surface sheet 104, it is preferable that, at the center in the lengthwise direction (center line CL), the widthwise side ends of the sheet member of t the top-surface sheet 104 provided on the skin side with respect to the skin-side core-wrapping sheet 108, which is the recycled part, are respectively located outside the widthwise side ends of the skin-side core-wrapping sheet 108. As shown in FIG. 6, in the diaper 100 of the present embodiment, the widthwise side ends of the top-surface sheet 104 are located outside the widthwise side ends of the skin-side core-wrapping sheet 108, on the center line CL in the lengthwise direction. That is, in the width direction, the skin-side core-wrapping sheet 108 is provided inside the top-surface sheet 104. As a result, at least in the width direction, the skin-side core-wrapping sheet 108, which is the recycled part, can be completely covered from the skin side, and this makes it likely to reduce the risk that the skin-side core-wrapping sheet 108 containing the recycled-derived component comes into direct contact with the wearer's skin.

Furthermore, in the diaper 100 of the present embodiment, the top-surface sheet 104 serves as the sheet member, but the combination of the top-surface sheet 104 and the side sheets 107 may serve as the sheet member. Similarly to the top-surface sheet 104, it is preferable that the side sheets 107 are also made of the thermoplastic resin fiber (polyethylene (PE), polypropylene (PP), etc.) such as nonwoven fabric. The diaper 100 of the present embodiment includes the liquid-permeable top-surface sheet 104 and the liquid-impermeable side sheet 107, as the sheet members are provided on the skin side with respect to the skin-side core-wrapping sheet 108. As the sheet members of the diaper 100, this allows the liquid-permeable top-surface sheet 104 to absorb the excrement, and in addition by providing the liquid-impermeable side sheets 107, even if the excrement has been absorbed by the diaper 100 during usage and has once reached the skin-side core-wrapping sheet 108, it is possible to reduce the risk that the excrement containing recycled-derived component returns to the wearer's skin.

The pH of the aqueous solution that is obtained by adding 100 mL of the ion exchange water to 10 g of the skin-side core-wrapping sheet 108 may be 5.5 or less. That is, the skin-side core-wrapping sheet 108, which has the recycled-derived component, may be acidic. Even in the case where the pH of the aqueous solution that is obtained by adding 100 mL of the ion exchange water to 10 g of the skin-side core-wrapping sheet 108 is 5.5 or less and there is a possibility of having negative effect on the wearer's skin, by using the top-surface sheet 104 provided on the skin side with respect to the skin-side core-wrapping sheet 108, it is possible to reduce the risk of direct contact between the skin-side core-wrapping sheet 108 and the wearer's skin. Further, during usage, there is a risk that the excrement that has been absorbed by the skin-side core-wrapping sheet 108 returns to the wearer's skin. At this time, there is a risk that the excrement in which the acidity has been increased by the skin-side core-wrapping sheet 108 returns to the skin side. In this regard, by providing the top-surface sheet 104 on the skin side with respect to the skin-side core-wrapping sheet 108, this makes it easier to reduce the amount of the excrement that returns to the wearer's skin from the skin-side core-wrapping sheet 108, making it possible to reduce the risk that the excrement with increased acidity comes into contact with the wearer's skin.

The pH of the aqueous solution that is obtained by adding 100 mL of the ion exchange water to 10 g of the skin-side core-wrapping sheet 108 may be between 3.0 and 5.0 (inclusive). Even in such a case, the top-surface sheet 104 provided on a skin side with respect to the skin-side core-wrapping sheet 108 can reduce the risk that the wearer's skin come into direct contact with the skin-side core-wrapping sheet 108a. In addition, even in the case where the pH of the aqueous solution that is obtained by adding 100 mL of the ion exchange water to 10 g of the skin-side core-wrapping sheet 108 is 5.0 or less and there is a possibility of having negative effect on the wearer's skin, if the pH is 3.0 or more, the alkaline included in the excrement that has been absorbed during usage makes it likely to neutralize the acidity of the skin-side core-wrapping sheet 108, making it likely to reduce the negative effects on the wearer's skin. In addition, regarding the alkaline of the excrement in a state where the diaper 100 has absorbed, it is similar to the absorbent pad 1 of the first embodiment.

The pH value of the skin-side core-wrapping sheet 108 can be measured in a similar method as described above. Note that the pH value of the skin-side core-wrapping sheet 108 may be measured for all of the skin-side core-wrapping sheet 108, or may be measured for a portion of the skin-side core-wrapping sheet 108. To measure the pH value, the aqueous solution is measured to which 100mL of the ion exchange water is added, for 10 g of the skin-side core-wrapping sheet 108. For example, if the weight of the skin-side core-wrapping sheet 108 is 1 g, measure the pH value of the aqueous solution to which 50mL of the ion exchange water is added is measured.

Further, the recycled-derived component of the skin-side core-wrapping sheet 108, which is the recycled part, has may be sulfuric acid or hypochlorous acid. These sulfuric acid or hypochlorous acid are components contained in the recycled pulp mainly because of being subjected to the recycling process. Even if the skin-side core-wrapping sheet 108 has sulfuric acid or hypochlorous acid as a recycled-derived component, the top-surface sheet 104 provided on the skin side with respect to the skin-side core-wrapping sheet 108 can reduce a risk of direct contact between the skin-side core-wrapping sheet 108 and the wearer's skin. In addition, due to the top-surface sheet 104 provided on the skin side with respect to the skin-side core-wrapping sheet 108, it is possible to reduce the risk that the excrement that has been absorbed once by the skin-side core-wrapping sheet 108 during usage returns to the skin side with the acidity of the excrement increased due to the liquid absorbent pulp of the first absorbent core 33a, and comes into contact with the wearer's skin.

The pH of the aqueous solution that is obtained by adding 100 mL of the ion exchange water to 10 g of the skin-side core-wrapping sheet 108 may be greater than 7.0. In other words, the skin-side core-wrapping sheet 108 may be alkaline. In the case where the pH of the aqueous solution that is obtained by adding 100 mL of the ion exchange water to 10 g of the skin-side core-wrapping sheet 108 is greater than 7.0, there is a possibility of having negative effect on the wearer's skin. Even in such a case, by using the top-surface sheet 104 provided on the skin side with respect to the skin-side core-wrapping sheet 108, it is possible to reduce the risk of direct contact between the skin-side core-wrapping sheet 108 and the wearer's skin. Further, during usage, there is a risk that the excrement that has been absorbed by the skin-side core-wrapping sheet 108 returns to the wearer's skin. At this time, there is a risk that the excrement in which the alkalinity has been increased by the skin-side core-wrapping sheet 108 returns to the skin side. In this regard, by providing the top-surface sheet 104 on the skin side with respect to the skin-side core-wrapping sheet 108, this makes it easier to reduce the amount of the excrement that returns to the wearer's skin from the skin-side core-wrapping sheet 108, making it possible to reduce the risk that the excrement with increased alkalinity comes into contact with the wearer's skin.

The recycled-derived components included in the skin-side core-wrapping sheet 108, which is the recycled part, may be calcium chloride or a nitrogen compound. Calcium chloride or the nitrogen compound are components contained in the recycled pulp or components derived from the excrement mainly because of being subject to the recycling process. Even if the skin-side core-wrapping sheet 108 has calcium chloride or a nitrogen compound as a recycled-derived component, the top-surface sheet 104 provided on the skin side with respect to the skin-side core-wrapping sheet 108 can reduce a risk that direct contact between the skin-side core-wrapping sheet 108 and the wearer's skin. In addition, even in the case where, due to the top-surface sheet 104 provided on the skin side with respect to the skin-side core-wrapping sheet 108, the excrement has been absorbed once by the skin-side core-wrapping sheet 108 during usage and the alkalinity increases due to the recycled-derived components of the skin-side core-wrapping sheet 108, it becomes likely to reduce the amount of the excrement that returns to the wearer's skin from the skin-side core-wrapping sheet 108, making it possible to reduce the risk that the excrement with increased alkalinity comes into contact with the wearer's skin.

In addition, in the case where the recycled-derived component contained in the skin-side core-wrapping sheet 108, which is the recycled part, is a nitrogen compound, it is preferable that the weight of nitrogen within the nitrogen compound in the skin-side core-wrapping sheet 108 (recycled part) is greater than 0 ppm and equal to or less than 50 ppm. In the case where the recycled-derived component is a nitrogen compound, the nitrogen compound is a component derived from the excrement that has been absorbed by the absorbent article before recycling. For example, it is decomposed products derived from proteins and/or amino acids, such as ammonia, that is, components derived from the excrement that has been absorbed by the absorbent article before recycling. The pH value of the liquid absorbent pulp that contains nitrogen compounds as a recycled-derived component shows the alkaline (pH greater than 7).

Even in the case where the skin-side core-wrapping sheet 108 has a nitrogen compound as a recycled-derived component, when the weight of nitrogen within the nitrogen compound in the skin-side core-wrapping sheet 108 is greater than 0 ppm and equal to or less than 50 ppm, it is possible to reduce negative effects on the wearer's skin compared to the case where the weight ratio of nitrogen within the nitrogen compound is greater than 50 ppm. In addition, even in the case where, due to the top-surface sheet 104 provided on the skin side with respect to the skin-side core-wrapping sheet 108, the excrement has been absorbed once by the skin-side core-wrapping sheet 108 during usage and the alkalinity increases due to the recycled-derived components of the skin-side core-wrapping sheet 108, it becomes likely to reduce the amount of the excrement that returns to the wearer's skin from the skin-side core-wrapping sheet 108, making it possible to reduce the risk that the excrement with increased alkalinity comes into contact with the wearer's skin. Note that the ratio of the weight of nitrogen within the nitrogen compound to the skin-side core-wrapping sheet 108 (recycled part) is obtained in the same manner as in the absorbent pad 1 of the first embodiment.

The recycled-derived component contained in the skin-side core-wrapping sheet 108, which is the recycled part, may be a decomposed product of superabsorbent polymer (SAP), the molecular weight of the decomposed product being less than 500, and in particular, may be sodium polyacrylate whose molecular weight is less than 500. In the case where the recycled-derived component is sodium polyacrylate (decomposed product of SAP) whose molecular weight is less than 500, the sodium polyacrylate (decomposed product of SAP) whose molecular weight is less than 500 is a decomposed product of the superabsorbent polymer (SAP) that is used in the absorbent article before recycling. It is sodium polyacrylate whose molecular weight has become less than 500 due to the decomposition of the superabsorbent polymer (SAP) in the recycling process of used absorbent articles.

In diaper 100, by providing the top-surface sheet 104 on the skin side with respect to the skin-side core-wrapping sheet 108, which is the recycled part, this reduces the risk of direct contact between the skin-side core-wrapping sheet 108 and the wearer's skin, making it possible to reduce the risk that the sodium polyacrylate whose molecular weight is less than 500 penetrates into the wearer's skin. In addition, even in the case where the excrement that has been absorbed once by the skin-side core-wrapping sheet 108 contains the sodium polyacrylate whose molecular weight is less than 500, it makes the absorbed excrement less likely to return to the skin side, and makes it possible to reduce the risk of negative effects on the wearer's skin.

Regarding the top-surface sheet 104 provided on the skin side with respect to the skin-side core-wrapping sheet 108, which is the recycled part, it is preferable that the adhesive portion 104s where an adhesive is applied is provided to the non-skin-side surface of the top-surface sheet 104 and that the adhesive is hydrophobic. Also, regarding this hydrophobic adhesive, it is more preferable that both ends of the adhesive in the lengthwise direction match both ends of the top-surface sheet 104 in the lengthwise direction, or both ends of the adhesive in the width direction match both ends of the top-surface sheet 104 in the width direction.

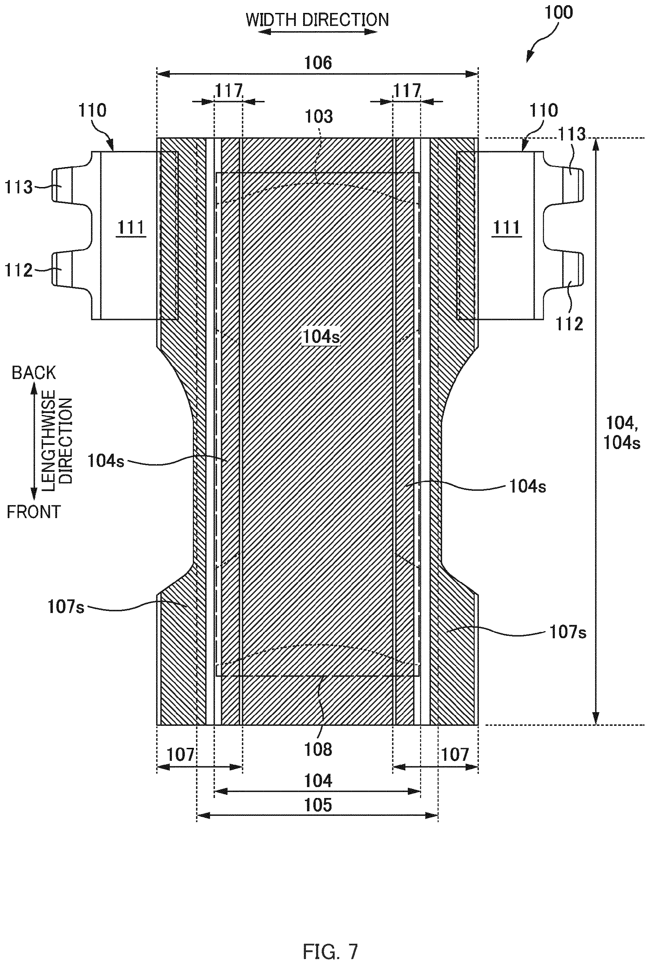

FIG. 7 is a diagram illustrating the adhesive portion 104s and the adhesive portion 107s. For convenience, the top-surface sheet 104, the adhesive portion 104s, and the adhesive portion 107s are shown with solid lines, and members such as the elastic members 171, 181, and 121 are omitted.

The diaper 100 of the present embodiment has the adhesive portion 104s in which an adhesive is applied to the non-skin-side surface of the top-surface sheet 104 and the top-surface sheet 104 and the core-wrapping sheet 108 adhere to each other. As shown in FIGS. 6 and 7, the adhesive portion 104s has a plurality of rectangular adhesive regions arranged side-by-side in the width direction, and both ends of the adhesive portion 104s in the lengthwise direction match both ends of the top-surface sheet 104 in the lengthwise direction. Further, both widthwise ends of the adhesive portion 104s are provided inside both widthwise ends of the top-surface sheet 104 and inside both widthwise ends of the skin-side core-wrapping sheet 108.

Even in the case where the excrement that has been absorbed by the diaper 100 during usage reaches the skin-side core-wrapping sheet 108, the hydrophobic adhesive provided between the top-surface sheet 104 and the skin-side core-wrapping sheet 108 makes it likely to suppress the excrement that is in contact with the recycled-derived component in the skin-side core-wrapping sheet 108 from returning to the wearer's skin. In addition, the larger the area of the adhesive provided in the non-skin-side surface of the top-surface sheet 104, the more the amount of the excrement returning to the wearer's skin from the skin-side core-wrapping sheet 108 can be reduced. Accordingly, the following configuration is more preferable: both lengthwise ends of the adhesive provided in the non-skin-side surface of the top-surface sheet 104 match both lengthwise ends of the top-surface sheet 104; or both widthwise ends of the adhesive match both widthwise ends of the top-surface sheet 104.

Further, a configuration is more preferable in which as shown in FIGS. 5 to 7, by applying the adhesive to the non-skin-side surface of the side sheets 107, the adhesive portions 107s are provided which adhere to a member located on the non-skin side of the side sheets 107 (on the back-surface sheet 105 in the diaper 100). It is preferred that the adhesive of the adhesive portion 107s is hydrophobic.

### Other embodiments

The above embodiments are simply to facilitate understanding of the present disclosure and are not in any way to be construed as limiting the present disclosure. The present disclosure may variously be changed or altered without departing from its gist and encompass equivalents thereof.

In the above embodiments, the absorbent pad 1 and the tape-type disposable diaper 100 for adults have been described as an example, but the present invention is not limited thereto. The absorbent article of the present invention is applicable to tape-type disposable diapers for children, underpants-shaped disposable diapers for adults or children, urine absorbing pads, sanitary napkins, shorts-shaped sanitary napkin, disposable diapers for pets, and the like.

In the above embodiments, in the absorbent pad 1, the absorbent cores 33a and 33b are the recycled part, and in the tape-type disposable diaper 2 for adults, the core-wrapping sheets 108 and 109 are the recycled part. But the present invention is not limited thereto. The core-wrapping sheets 38, 39, 41, and 42 of the absorbent pad 1 may be the recycled part, and the absorbent core 103 of the diaper 2 may be the recycled part. Further, in the absorbent pad 1, the absorbent cores 33 and 33b and the core-wrapping sheets 38, 39, 41, and 42 both may be the recycled part. In the diaper 2, the absorbent core 3 and the core-wrapping sheets 108 and 109 both may be the recycled part.

Further, of the absorbent article (absorbent pad 1, diaper 100), the recycled part which has the recycled pulp is not limited to the absorbent core and the core-wrapping sheet, but may be any member of the absorbent article. Even if any member is the recycled part, providing the sheet member on the skin side with respect to the recycled part can reduce the risk of the recycled part coming into contact with the wearer's skin, making it possible to reduce the risk that the recycled-derived component contained in the recycled part have negative effects on the wearer's skin.

Further, the sheet member provided on the skin side with respect to the recycled part is not limited to the core-wrapping sheet, the top-surface sheet, and the side sheets. Desired members can be added, for example, providing such as a separate sheet-like member, as long as it is provided on the skin side with respect to the recycled part.

### REFERENCE SIGNS LIST

1 absorbent pad (absorbent article),
33 absorbent body,
33a first absorbent core (absorbent core, recycled part),
33b second absorbent core (absorbent core, recycled part),
33c third absorbent core (sheet member),
34 top-surface sheet (sheet member),
35 back-surface sheet, 36 exterior sheet,
37 side sheet (sheet member),
38 first core-wrapping sheet (sheet member),
38s adhesive portion (adhesive portion to which adhesive is applied),
39 second core-wrapping sheet,
41 third core-wrapping sheet,
42 fourth core-wrapping sheet,
45a, 45b slit, 46 pair of compressed portions, 48 leak-proof wall,
60 leak-proof-wall elastic member,
100 diaper (absorbent article),
101A front waist portion, 101B crotch portion, 101C back waist portion,
103 absorbent core,
104 top-surface sheet,
104s adhesive portion (adhesive portion to which adhesive is applied),
105 back-surface sheet, 106 exterior sheet,
107 side sheet,
107s adhesive portion (adhesive portion to which adhesive is applied),
108 skin-side core-wrapping sheet (core-wrapping sheet, recycled part),
109 non-skin-side core-wrapping sheet (core-wrapping sheet, recycled part),
110 flap portion, 111 base sheet,
112 first fastening member, 113 second fastening member,
114 target portion,
116 leg elastic member,
117 inner leak-proof gather, 118 outer leak-proof gather,
121 waist elastic member, 122 waist gather,
171 inner leak-proof elastic member, 181 outer leak-proof elastic member

## Claims

1. An absorbent article having a longitudinal direction, a lateral direction, and a thickness direction that are orthogonal to each other,
the absorbent article comprising:
a recycled part containing recycled pulp that has been obtained by making a used disposable absorbent article be subject to a recycling process; and
a sheet member that overlaps at least a part of the recycled part as viewed in the thickness direction,
the recycled part containing a recycled-derived component,
the sheet member being provided on a skin side with respect to the recycled part.

2. The absorbent article according to claim 1, wherein
the recycled part is an absorbent core that absorbs liquid,
the absorbent core has liquid absorbent pulp,
the liquid absorbent pulp has the recycled pulp that has at least the recycled-derived component, and
a pH of an aqueous solution that is obtained by adding 100 mL of an ion exchange water to 10 g of the liquid absorbent pulp is 5.5 or less.

3. The absorbent article according to claim 2, wherein
the pH of the aqueous solution that is obtained by adding 100 mL of the ion exchange water to 10 g of the liquid absorbent pulp is between 3.0 and 5.0 (inclusive).

4. The absorbent article according to claim 1, wherein
the recycled part is a core-wrapping sheet that covers at least a part of an absorbent core that absorbs liquid, and
a pH of an aqueous solution that is obtained by adding 100 mL of an ion exchange water to 10 g of the core-wrapping sheet is 5.5 or less.

5. The absorbent article according to claim 4, wherein
the pH of the aqueous solution that is obtained by adding 100 mL of the ion exchange water to 10 g of the core-wrapping sheet is between 3.0 and 5.0 (inclusive).

6. The absorbent article according to any one of claims 1 to 5, wherein
the recycled-derived component is either of a sulfuric acid and a hypochlorous acid.

7. The absorbent article according to claim 1, wherein
the recycled part is an absorbent core that absorbs liquid,
the absorbent core has liquid absorbent pulp,
the liquid absorbent pulp has the recycled pulp that has at least the recycled-derived component, and
a pH of an aqueous solution that is obtained by adding 100 mL of an ion exchange water to 10 g of the liquid absorbent pulp is greater than 7.0.

8. The absorbent article according to claim 7, wherein
the absorbent core also has superabsorbent polymer containing sodium acrylate.

9. The absorbent article according to claim 1, wherein
the recycled part is a core-wrapping sheet that covers at least a part of an absorbent core that absorbs liquid, and
a pH of an aqueous solution that is obtained by adding 100 mL of an ion exchange water to 10 g of the core-wrapping sheet is 7.0 or more.

10. The absorbent article according to any one of claims 1 and 7 to 9, wherein
the recycled-derived component is a nitrogen compound, and
a weight of nitrogen within the nitrogen compound in the recycled part is greater than 0 ppm and equal to or less than 50 ppm.

11. The absorbent article according to any one of claims 1 and 7 to 10, wherein
the recycled-derived component is either of calcium chloride and a nitrogen compound.

12. The absorbent article according to any one of claims 1 to 11, wherein
an adhesive is provided in a non-skin-side surface of the sheet member, and
the adhesive is hydrophobic.

13. The absorbent article according to claim 12, wherein
either of followings is satisfied:
both ends of the adhesive in the longitudinal direction are match both ends of the sheet member in the longitudinal direction; and
both ends of the adhesive in the lateral direction match both ends of the sheet member in the lateral direction.

14. The absorbent article according to any one of claims 1 to 13, wherein
the sheet member is made of a thermoplastic resin fiber, and
the sheet member is provided so as to be able to come into contact with a wearer's skin.

15. The absorbent article according to claim 14, wherein
the sheet member includes a liquid-permeable sheet portion and a liquid-impermeable sheet portion.

16. The absorbent article according to any one of claims 1 to 15, wherein
at a center in the longitudinal direction,
lateral side ends of the sheet member are located outside corresponding lateral side ends of the recycled part.

17. The absorbent article according to any one of claims 1 to 16, wherein
the recycled-derived component has a sodium polyacrylate a molecular weight of which is less than 500.
